(19) Europäisches Patentamt
European Patent Office
Office européen des brevets

(11) **EP 2 277 882 A1**

(12) **DEMANDE DE BREVET EUROPEEN**

(43) Date de publication:
**26.01.2011 Bulletin 2011/04**

(51) Int Cl.:
***C07D 491/04*** *(2006.01)* ***A61K 31/407*** *(2006.01)*

(21) Numéro de dépôt: **10290411.7**

(22) Date de dépôt: **20.07.2010**

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**
Etats d'extension désignés:
**BA ME RS**

(30) Priorité: **21.07.2009 FR 0903572**

(71) Demandeur: **Les Laboratoires Servier**
**92284 Suresnes Cedex (FR)**

(72) Inventeurs:
- **de Nanteuil, Guillaume**
  **92150 Suresnes (FR)**
- **Cimetiere, Bernard**
  **75012 Paris (FR)**
- **Dekeyne, Anne**
  **78720 Cernay la Ville (FR)**
- **Millan, Mark**
  **78230 Le Pecq (FR)**

(54) **Composés pipéridiniques, leur procédé de préparation et les compositions pharmaceutiques qui les contiennent**

(57) L'invention concerne les composés de formule (I) :

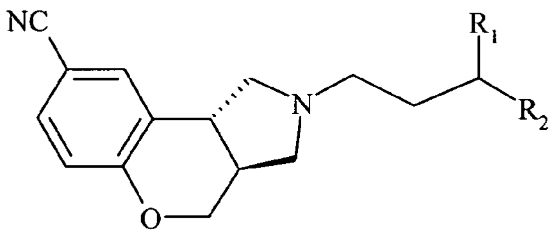

(I)

dans laquelle $R_1$ et $R_2$ forment ensemble la chaîne carbonée suivante :

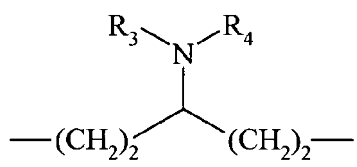

dans laquelle :
➢ $R_3$ représente un atome d'hydrogène ou un groupement alkyle,
➢ $R_4$ représente un atome d'hydrogène, un groupement alkyle, aryle, hétéroaryle, 3,4-dioxocyclobutenyle, alkylcarbonyle, cycloalkylcarbonyle, hétérocycloalkylcarbonyle, benzoyle, arylsulfonyle ou hétéroarylsulfonyle, chacun de ces groupements étant éventuellement substitué,
➢ $R_3$ et $R_4$ forment ensemble avec l'atome d'azote qui les porte un cycle comportant de 5 à 8 chaînons, le cycle ainsi formé pouvant être éventuellement substitué.

EP 2 277 882 A1

## Description

La présente invention concerne de nouveaux dérivés chroméniques, leur procédé de préparation, et les compositions pharmaceutiques qui les contiennent.

Il est bien établi que les voies dopaminergiques se projetant sur les structures limbiques et le cortex frontal jouent des rôles importants dans le contrôle de l'humeur, dans les phénomènes de récompense, dans la fonction motrice et dans la cognition. Les récepteurs dopaminergiques $D_3$ sont présents en forte concentration dans ces structures corticales et limbiques comme le noyau accumbens, le globus pallidus, le thalamus et le cortex frontal, tandis que leur densité est relativement faible dans le striatum. En conséquence, ils sont une cible de choix pour les médicaments psychotropes (Psychopharmacology, 1998, 135, 1-16 ; CNS Neurol. Disord. Drug Targets, 2006, 5, 25-43).

Le blocage des récepteurs $D_2$ provoque une amélioration des symptômes positifs mais il est aussi associé à la catalepsie, à une diminution de la fonction cognitive, et aux effets pouvant induire une dépression (CNS Drug Discov., 2006, 1, 271-88 ; Drug Discov. Today, 2005, 10, 917-25). Or, bien que son impact sur les symptômes positifs ne soit pas définitivement démontré, le blocage des récepteurs $D_3$ influence favorablement l'humeur, améliore la fonction cognitive, et s'oppose à la catalepsie (Thérapie 2008, 63, 187-229).

Ces observations suggèrent qu'un composé possédant un profil optimisé, *via* une activité antagoniste préférentielle pour les récepteurs $D_3$ et une activité antagoniste adéquate pour les récepteurs $D_2$, pourrait se placer dans une « fenêtre thérapeutique » idéale pour un contrôle optimum de l'ensemble des symptômes de la schizophrénie tout en s'affranchissant des effets secondaires extrapyramidaux (catalepsie) et autres inconvénients liés à un blocage très sélectif de chacun des récepteurs dopaminergiques (Drug Discov. Today, 2005, 10, 917-25 ; Thérapie 2008, 63, 187-229).

A partir de ces observations et de divers résultats documentés dans la littérature, on peut comprendre qu'une préférence pour les récepteurs $D_3$ *vs.* $D_2$ confère aux produits de l'invention un intérêt majeur pour une utilisation comme médicament dans le traitement de la schizophrénie et d'autres psychoses (Drug Discov. Today, 2005, 10, 917-25 ; Neurosci. Behav. Rev., 2001, 25, 427-43), de l'abus de drogues, y compris le « récidivisme » (Brain Res. Rev., 2005, 49, 77-105 ; J. Med. Chem., 2005, 48, 3664-79) : par exemple avec les psychostimulants, cocaïne et amphétamine (Int. J. Neuropsychopharmacol., 2007, 10, 167-81 ; J. Pharmacol. Exp. Ther., 2007, 321, 573-82), la nicotine (Neuropsychopharmacol., 2003, 28, 1272-80 ; Int. J. Neuropsychopharmacol., 2006, 9, 585-602), les opiacés (Synapse, 2003, 48, 154-6 ; Psychopharmacology, 2004, 175, 127-33), et l'éthanol (Pharmacol. Biochem. Behav., 2005, 81, 190-7 ; FASEB J., 2007, 20, 2223-33). Les produits de l'invention sont aussi susceptibles d'être utilisés dans le traitement des troubles provoqués par le stress comme les états anxieux et la toxicomanie (Psychopharmacology, 2004, 176, 57-65 ; Prog. Neurobiol., 2003, 70, 83-244), le traitement des états dépressifs, unipolaires et bipolaires (Eur. Neuropsychopharmacol., 2008, 18, 271-7 ; Mol. Interv., 2008, 8, 230-41), le traitement des comportements impulsifs comme les troubles obsessionnels compulsifs (Psychiatry Res., 2003, 119, 1-10 ; Am. J. Med. Genet. B Neuropsychiatr. Genet., 2006, 141B, 409-13), et l'agressivité (J. Neural. Transm., 2003, 110, 561-72), le traitement de la maladie de Parkinson, en administration seule ou en association avec les agonistes dopaminergiques ou le L-DOPA (Neurobiol. Dis., 2009, sous presse ; Exp. Neurol., 2004, 188, 128-38), le traitement du tremblement essentiel (PNAS, 2006, 103, 10753-8 ; Brain, 2007, 130, 1456-64), des troubles de la mémoire et d'autres troubles cognitifs associés aux maladies psychiatriques et neurologiques, comme les démences et la maladie d'Alzheimer (Psychopharmacology, 2005, 179, 567-75 ; J. Neurochem., 2007, 100, 1047-61), des troubles développementaux chez l'enfant ou l'adolescent, comme le trouble du spectre autistique et le trouble déficitaire de l'attention avec hyperactivité (Biol. Psychiatry, 2008, 65, 625-30), le traitement de la douleur, par exemple en association avec les opiacés (Psychopharmacology, 1999, 144, 239-47 ; Prog. Neurobiol., 2002, 66, 355-474), ou encore des nausées provoquées, par exemple, par les cytotoxiques et les agonistes dopaminergiques (J. Neural Transm., 1999, 105, 1045-61 ; Eur. J. Pharmacol., 1996, 301, 143-9). Les composés de l'invention sont aussi utiles pour le traitement de l'éjaculation prématurée (J. Sex. Med. 2009, 6, 980-8 ; Br. J. Pharmacol. 2008, 154, 1150-9) ainsi que dans la protection rénale, par exemple associée au diabète ou à un traitement chronique avec un antipsychotique perturbant le métabolisme (Lab. Investigation, 2006, 86, 262-74 ; Naunyn Schmiedebergs Arch. Pharmacol., 2005, 371, 420-7).

Les composés de cette invention, outre le fait qu'ils soient nouveaux, possèdent des propriétés particulièrement intéressantes, en se fixant de manière puissante et préférentielle aux récepteurs dopaminergiques $D_3$.

Plus spécifiquement, la présente invention concerne les composés de formule (I) :

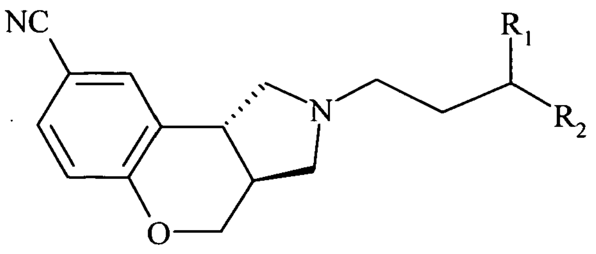

(I)

dans laquelle $R_1$ et $R_2$ forment ensemble la chaîne carbonée suivante :

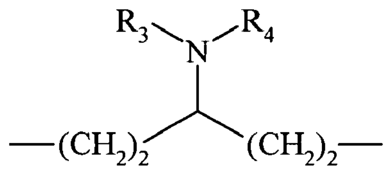

dans laquelle :

➢ $R_3$ représente un atome d'hydrogène ou un groupement alkyle ($C_1$-$C_6$) linéaire ou ramifié,

➢ $R_4$ représente :

- un atome d'hydrogène,
- un groupement alkyle ($C_1$-$C_6$) linéaire ou ramifié, aryle, hétéroaryle ou 3,4-dioxocyclobutenyle, chacun de ces groupements étant éventuellement substitué par un ou plusieurs groupements, identiques ou différents, choisis parmi halogène ; alkyle ($C_1$-$C_6$) linéaire ou ramifié ; alkylcarbonyle ($C_1$-$C_6$) linéaire ou ramifié ; carboxy ; hydroxy ; cyano ; nitro ; aminocarbonyle non-substitué ou substitué par un ou plusieurs groupements alkyle ($C_1$-$C_6$) linéaire ou ramifié ; amino non-substitué ou substitué par un ou deux groupements alkyle ($C_1$-$C_6$) linéaire ou ramifié,
- un groupement -$COR_5$,
- un groupement -$SO_2R_5$,

➢ $R_5$ représente un groupement alkyle ($C_1$-$C_6$) linéaire ou ramifié, cycloalkyle ($C_3$-$C_8$), hétérocycloalkyle, aryle ou hétéroaryle, chacun de ces groupements étant éventuellement substitué par un ou plusieurs groupements, identiques ou différents, choisis parmi halogène ; alkyle ($C_1$-$C_6$) linéaire ou ramifié ; alkylcarbonyle ($C_1$-$C_6$) linéaire ou ramifié ; carboxy ; hydroxy ; cyano ; nitro ; aminocarbonyle non-substitué ou substitué par un ou plusieurs groupements alkyle ($C_1$-$C_6$) linéaire ou ramifié ; amino non-substitué ou substitué par un ou deux groupements alkyle ($C_1$-$C_6$) linéaire ou ramifié,

➢ ou bien, $R_3$ et $R_4$ forment ensemble avec l'atome d'azote qui les porte un cycle comportant de 5 à 8 chaînons, le cycle ainsi défini pouvant être éventuellement substitué par un ou plusieurs groupements, identiques ou différents, choisis parmi halogène ; alkyle ($C_1$-$C_6$) linéaire ou ramifié ; hydroxy ; oxo ; amino non-substitué ou substitué par un ou deux groupements alkyle ($C_1$-$C_6$) linéaire ou ramifié,

leurs isomères de position, leurs énantiomères, leurs diastéréoisomères, ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, acétique, trifluoroacétique, lactique, malonique, succinique, glutamique, fumarique, maléique, citrique, oxalique, méthane sulfonique, benzène sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Par groupement aryle, on entend un groupement phényle ou naphtyle.

Par groupement hétéroaryle, on entend un groupement monocyclique ou bicyclique dans lequel au moins un

des cycles est aromatique, comportant de 5 à 11 chaînons et de 1 à 4 hétéroatomes, choisis parmi l'azote, l'oxygène et le soufre.

Le terme hétérocycloalkyle représente un groupement mono ou bicyclique, non aromatique, contenant de 4 à 11 chaînons et possédant de 1 à 4 hétéroatomes choisis parmi azote, oxygène et soufre.

Les groupements préférés aryle sont le groupement phényle.

Les groupements préférés hétéroaryle sont les groupements pyridinyle et pyrimidinyle.

Les groupements préférés hétérocycloalkyle sont le groupement azétidine.

Dans les composés de formule (I), $R_3$ représente préférentiellement un atome d'hydrogène ou un groupement méthyle.

De manière avantageuse, les composés de formule (I) sont les composés pour lesquels $R_4$ représente un atome d'hydrogène ou un groupement alkyle ($C_1$-$C_6$) linéaire ou ramifié.

Des composés préférés de l'invention sont ceux pour lesquels $R_4$ représente le groupement -$COR_5$, où $R_5$ est défini tel que précédemment.

D'autres composés préférés de l'invention sont ceux pour lesquels $R_4$ représente le groupement -$SO_2R_5$, où $R_5$ est défini tel que précédemment.

Le groupement $R_5$ représente préférentiellement le groupement alkyle ($C_1$-$C_6$) linéaire ou ramifié, le groupement cycloalkyle ($C_3$-$C_8$), le groupement aryle ou le groupement hétéroaryle.

Plus particulièrement, les composés de formule (I) préférés sont les composés pour lesquels $R_4$ représente -$COR_5$ où $R_5$ est un groupement alkyle ($C_1$-$C_6$) linéaire ou ramifié.

Une autre possibilité préférée pour les composés de formule (I) consiste en ce que $R_4$ représente -$COR_5$ où $R_5$ est un groupement cycloalkyle ($C_3$-$C_8$), éventuellement substitué.

De manière préférée, les composés de formule (I) sont les composés pour lesquels $R_4$ représente -$COR_5$ où $R_5$ est un groupement aryle, éventuellement substitué.

Plus particulièrement, les composés de formule (I) sont les composés pour lesquels $R_4$ représente -$SO_2R_5$ où $R_5$ est un groupement aryle ou hétéroaryle, éventuellement substitué.

Une autre possibilité avantageuse consiste en ce que $R_3$ et $R_4$ peuvent former ensemble avec l'atome d'azote qui les porte un cycle à 5 chaînons, le cycle ainsi formé pouvant être éventuellement substitué.

Les composés préférés de l'invention sont le :

- (3a*S*,9b*R*)-2-[2-(*trans*-4-aminocyclohexyl)éthyl]-1,2,3,3a,4,9b-hexahydrochroméno [3,4-*c*]pyrrole-8-carbonitrile ;
- (3a*S*,9b*R*)-2-{2-[*trans*-4-(méthylamino)cyclohexyl]éthyl}-1,2,3,3a,4,9b-hexahydro chroméno[3,4-*c*]pyrrole-8-carbonitrile ;
- (3a*S*,9b*R*)-2-{2-[*trans*-4-(diméthylamino)cyclohexyl]éthyl}-1,2,3,3a,4,9b-hexahydrochroméno[3,4-*c*]pyrrole-8-carbonitrile ;
- *N*-(*trans-4*-{2-[(3a*S*,9b*R*)-8-cyano-1,3a,4,9b-tétrahydrochroméno[3,4-*c*]pyrrol-2(3*H*)-yl]éthyl} cyclohexyl)acétamide ;
- *N*-(*trans*-4-{2-[(3a*S*,9b*R*)-8-cyano-1,3a,4,9b-tétrahydrochroméno[3,4-*c*]pyrrol-2(3*H*)-yl]éthyl}cyclohexyl)-2,2-diméthylpropanamide ;
- *N*-(*trans*-4-{2-[(3a*S*,9b*R*)-8-cyano-1,3a,4,9b-tétrahydrochroméno[3,4-*c*]pyrrol-2(3*H*)-yl]éthyl} cyclohexyl)-*N*-méthylacétamide ;
- *N*-(*trans*-4-{2-[(3a*S*,9b*R*)-8-cyano-1,3a,4,9b-tétrahydrochroméno[3,4-*c*]pyrrol-2(3*H*)-yl]éthyl}cyclohexyl) cyclobutanecarboxamide ;
- *N*-(*trans*-4-{2-[(3a*S*,9b*R*)-8-cyano-1,3a,4,9b-tétrahydrochroméno[3,4-*c*]pyrrol-2(3*H*)-yl]éthyl}cyclohexyl) cyclopropanecarboxamide ;
- *N*-(*trans*-4-{2-[(3a*S*,9b*R*)-8-cyano-1,3a,4,9b-tétrahydrochroméno[3,4-*c*]pyrrol-2(3*H*)-yl]éthyl}cyclohexyl)-3,3-difluorocyclobutanecarboxamide ;
- *cis*-*N*-(*trans*-4-2-[(3a*S*,9b*R*)-8-cyano-1,3a,4,9b-tétrahydrochroméno[3,4-*c*]pyrrol-2(3*H*)-yl]éthyl}cyclohexyl)-3-hydroxycyclobutanecarboxamide ;
- *N*-(*trans*-4-{2-[(3a*S*,9b*R*)-8-cyano-1,3a,4,9b-tétrahydrochroméno[3,4-*c*]pyrrol-2(3*H*)-yl]éthyl} cyclohexyl)-*N*-méthylcyclobutanecarboxamide ;
- N-(*trans*-4-{2-[(3a*S*,9b*R*)-8-cyano-1,3a,4,9b-tétrahydrochroméno[3,4-*c*]pyrrol-2(3*H*)-yl]éthyl}cyclohexyl) benzamide ;
- 4-chloro-*N*-(*trans*-4-{2-[(3a*S*,9b*R*)-8-cyano-1,3a,4,9b-tétrahydrochroméno [3,4-*c*]pyrrol-2(3*H*)-yl]éthyl}cyclohexyl) benzamide ;
- N-(*trans*-4-12-[(3a*S*,9b*R*)-8-cyano-1,3a,4,9b-tétrahydrochroméno[3,4-*c*]pyrrol-2(3*H*)-yl]éthyl}cyclohexyl)-4-fluorobenzènesulfonamide ;
- *N*-(*trans*-4-{2-[(3a*S*,9b*R*)-8-cyano-1,3a,4,9b-tétrahydrochroméno[3,4-c]pyrrol-2(3*H*)-yl]éthyl}cyclohexyl)-3-pyridinesulfonamide ;

- 4-chloro-*N*-(*trans*-4-{2-[(3a*S*,9b*R*)-8-cyano-1,3a,4,9b-tétrahydrochroméno [3,4-*c*]pyrrol-2(3*H*)-yl]éthyl}cyclohexyl) benzènesulfonamide ;
- (3a*S*,9b*R*)-2-{2-[*trans*-4-(2-oxopyrrolidin-1-yl)cyclohexyl]éthyl}-1,2,3,3a,4,9b-hexahydrochroméno[3,4-*c*]pyrrole-8-carbonitrile.

Les sels d'addition à un acide ou une base pharmaceutiquement acceptable des composés préférés de l'invention font partie intégrante de l'invention.

L'invention s'étend également au procédé de préparation des composés de formule (I), **caractérisé en ce que** l'on utilise comme produit de départ un composé de formule (II) :

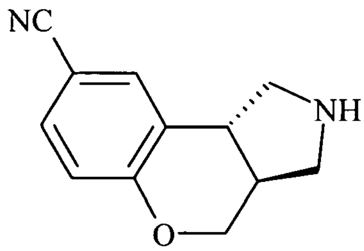

(II)

qui subit une réaction d'amination réductrice en présence d'un agent réducteur, tel que le triacétoxyborohydrure ou le cyanoborohydrure de sodium, et d'un composé de formule (III) :

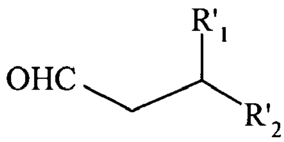

(III)

dans laquelle $R'_1$ et $R'_2$ forment ensemble la chaîne carbonée suivante :

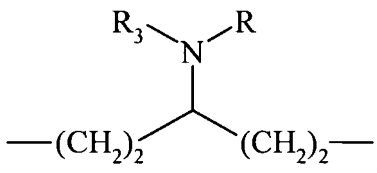

dans laquelle $R_3$ est tel que défini précédemment et R représente un groupement protecteur de la fonction amine, comme par exemple le groupement *tert*-butyloxycarbonyle, pour conduire au composé de formule (IV) :

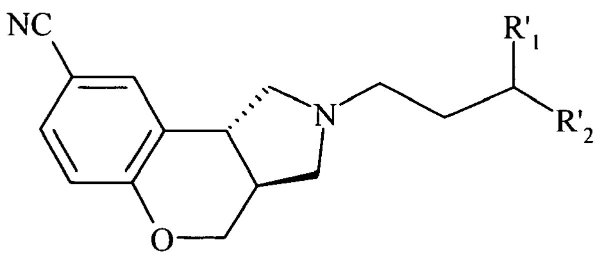

(IV)

dans laquelle $R'_1$ et $R'_2$ sont tels que définis précédemment, qui est ensuite soumis à une réaction de déprotection de la fonction amine, par exemple en présence d'acide trifluoroacétique, pour conduire au composé de formule (I/a), cas particulier des composés de formule (1) :

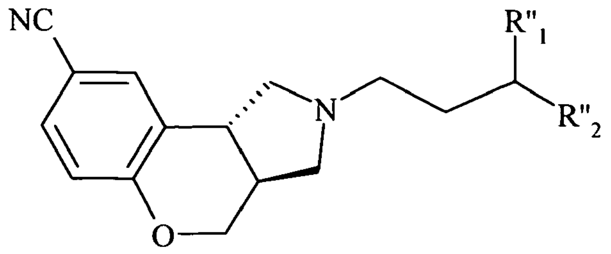

(I/a)

dans laquelle R"$_1$ et R"$_2$ forment ensemble la chaîne carbonée suivante :

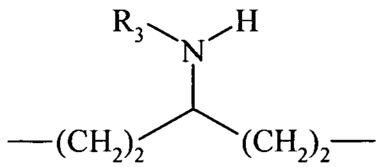

dans laquelle $R_3$ est tel que défini précédemment,
composé de formule (I/a) qui est ensuite soumis, si nécessaire :

■ **soit** à une réaction d'amination réductrice avec un agent réducteur, tel que le triacétoxyborohydrure ou le cyanoborohydrure de sodium, en présence d'un composé de formule (V) :

R'-CHO (V)

dans laquelle R' représente un atome d'hydrogène ou un groupement alkyle ($C_1$-$C_5$) linéaire ou ramifié ;

■ **soit** à l'action d'un composé de formule (VI) :

R"-Y (VI)

dans laquelle Y représente un atome d'halogène, un groupement hydroxy ou alkoxy ($C_1$-$C_6$) linéaire ou ramifié et R" représente un groupement aryle, hétéroaryle, 3,4-dioxocyclobutenyle, -$COR_5$, -$SO_2R_5$, où $R_5$ est tel que défini précédemment, pour conduire au composé de formule (I/b), cas particulier des composés de formule (I) :

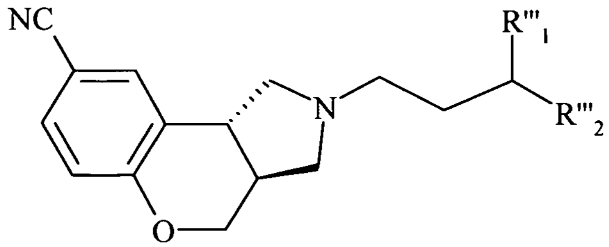

(I/b)

dans laquelle R"'$_1$ et R"'$_2$ forment ensemble la chaîne carbonée suivante :

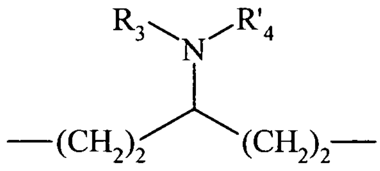

dans laquelle $R_3$ est tel que défini précédemment et $R'_4$ représente un groupement alkyle ($C_1$-$C_6$) linéaire ou ramifié, aryle, hétéroaryle, 3,4-dioxocyclobutenyle, $-COR_5$ ou $-SO_2R_5$, où $R_5$ est tel que défini précédemment, une variante dans la préparation du composé de formule (I/b) consistant, une fois l'étape de couplage sur le composé de formule (I/a) réalisée, en l'utilisation des réactions classiques de chimie afin de modifier, dans un deuxième temps, les substituants du composé de formule (VI),

les composés de formule (I/a) et (I/b), qui constituent l'ensemble des composés de formule (I), pouvant être ensuite purifiés selon une technique classique de séparation, que l'on transforme, si on le souhaite, en ses sels d'addition à un acide ou à une base pharmaceutiquement acceptable et dont on sépare éventuellement les isomères, s'ils existent, selon une technique classique de séparation.

Les composés de formule (II), (III), (V) et (VI) sont commerciaux ou aisément accessibles à l'homme du métier par des réactions de chimie classiques ou décrites dans la littérature.

L'invention s'étend également aux compositions pharmaceutiques renfermant comme principe actif au moins un composé de formule (I) seul ou en combinaison avec un ou plusieurs excipients ou véhicules inertes non toxiques. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les comprimés sublinguaux, les gélules, les tablettes, les suppositoires, les crèmes, les pommades, les gels dermiques, les préparations injectables, et les suspensions buvables.

La posologie utile varie selon l'âge et le poids du patient, la nature et la sévérité de l'affection ainsi que la voie d'administration. Celle-ci peut être nasale, rectale, parentérale ou orale. D'une manière générale, la posologie unitaire s'échelonne entre 1 et 500 mg pour un traitement en 1 à 3 prises par 24 heures.

Les exemples suivants illustrent l'invention et ne la limitent en aucune façon. Les structures des composés décrits ont été confirmées par les techniques spectroscopiques usuelles.

Les préparations décrites ci-dessous conduisent à des produits de départ utilisés lors de la synthèse des composés de l'invention.

**Préparation 1 : *trans*-{4-[(tert-butoxycarbonyl)amino]cyclohexyl}acétate de méthyle**

A un mélange de 3,03 g de *trans*-(4-amino-cyclohexyl)acétate d'éthyle (17,7 mmol), obtenu en 7 étapes à partir du bicyclo[2.2.2]oct-5-èn-2-carbonitrile, et de 4,25 g de dicarbonate de di-*tert*-butyle (19,54 mmol) dans 60 ml de dichlorométhane, est ajouté 6,2 ml de triéthylamine. Le mélange est agité 2 heures à température ambiante. 50 ml d'une solution saturée en bicarbonate de sodium sont ajoutés. La solution est extraite avec 3 x 20 ml de dichlorométhane, lavée à la saumure, séchée ($MgSO_4$) et évaporée pour conduire au produit du titre sous la forme d'une poudre blanche.

*Point de fusion : 78 °C*

**Préparation 2 : *trans*-{4-[(*tert*-butoxycarbonyl)amino]cyclohexyl}acétaldéhyde**

A une solution de 1,78 g du composé de la Préparation 1 (6,6 mmol) dans 35 ml de toluène, est ajouté, à -78 °C, au goutte à goutte, 11 ml d'une solution de DIBAL-H 1 N dans l'hexane. Le mélange est agité 10 minutes à -78 °C puis traité avec 1,08 ml de méthanol dans 2 ml de toluène (goutte à goutte). On laisse remonter à température ambiante et on ajoute rapidement, au goutte à goutte, 47 ml d'une solution aqueuse saturée en tartrate double de sodium et de potassium (sel de Seignette). Après 1 heure d'agitation, la solution est extraite à l'éther, lavée à l'eau, séchée ($MgSO_4$) et évaporée pour conduire au produit du titre sous la forme d'un solide blanc.

*Point de fusion : 61-63 °C*

**Préparation 3 : *trans*-{4-[(*tert*-butoxycarbonyl)(méthyl)amino]cyclohexyl}acétate de méthyle**

A une solution de 4,28 g du composé de la Préparation 1 (15,8 mmol) dans 45 ml de DMF, est ajouté 1,38 ml de iodure de méthyle puis 884 mg d'hydrure de sodium. Après 18 heures d'agitation, le mélange est dilué dans l'éther et de l'eau puis une solution d'HCl 0,1 N est ajoutée jusqu'à pH = 3. La solution est extraite à l'éther, lavée à l'eau,

séchée ($MgSO_4$) puis évaporée. Une purification par chromatographie sur colonne de silice avec comme éluant un mélange cyclohexane/acétate d'éthyle (90 / 10) conduit au produit du titre sous forme d'une huile incolore.

**Préparation 4 : *trans*-{4-[(*tert*-butoxycarbonyl)(méthyl)amino]cyclohexy} acétaldéhyde**

Le produit du titre est obtenu selon le procédé décrit dans la Préparation 2 en prenant comme produit de départ le produit décrit dans la Préparation 3.

**Préparation 5 : (*trans*-4-12-[(3a*S*,9b*R*)-8-cyano-1,3a,4,9b-tétrahydrochroméno [3,4-*c*]pyrrol-2(3*H*)-yl]éthyl}cyclohexyl)carbamate** de ***tert*-butyle**

Dans 30 ml de dichlorométhane, sont ajoutés successivement 1,08 g de (3a*S*,9b*R*)-1,2,3,3a,4,9b-hexahydrochroméno[3,4-*c*]pyrrole-8-carbonitrile (synthétisé d'après Bioorg. Med. Chem. Lett. 1999, 9, 2059-2064) (5,42 mmol), 1,58 g du composé de la Préparation 2 (6,5 mmol) et 1,61 g de triacétoxyborohydrure de sodium (7,59 mmol). Le milieu réactionnel est agité 1 nuit à température ambiante. La solution est ensuite lavée avec une solution de soude 1 N puis avec de la saumure, séchée ($MgSO_4$) et évaporée. Une purification sur colonne de silice avec comme éluant un mélange dichlorométhane/méthanol/ammoniaque (99 / 1 / 0,1) fournit le produit du titre sous forme d'une poudre blanche.

**Préparation 6 : (*trans*-4-{2-[(3a*S*,9b*R*)-8-cyano-1,3a,4,9b-tétrahydrochroméno [3,4-*c*]pyrrol-2(3*H*)-yl]éthyl}cyclohexyl)méthylcarbamate de *tert*-butyle**

Le produit du titre est obtenu selon le procédé décrit dans la Préparation 5 en prenant comme produit de départ le composé décrit dans la Préparation 4 à la place du produit décrit dans la Préparation 2.

**Préparation 7 : Ester monométhylique de l'acide phtalique**

Le phtalate de diméthyle (15,73 mmol) est agité 24 heures à température ambiante dans 60 ml d'éthanol avec 15,73 ml d'une solution de soude 1 N. Le solvant est évaporé sous pression réduite à 40 °C puis le mélange réactionnel est dilué dans l'eau avant de ramener le pH à 3. Le milieu est extrait à l'acétate d'éthyle, séché ($MgSO_4$) et évaporé pour conduire au produit du titre sous forme d'une huile.

**EXEMPLE 1 : Dichlorhydrate de (3a*S*,9b*R*)-2-[2-(*trans*-4-aminocyclohexyl)éthyl]-1,2,3,3a,4,9b-hexahydrochroméno[3,4-*c*]pyrrole-8-carbonitrile**

A une solution de 1,5 g du composé de la Préparation 5 (3,5 mmol) dans 30 ml de dichlorométhane, est ajouté 2,9 ml d'acide trifluoroacétique. Le mélange est agité 4,5 heures à température ambiante et le solvant est évaporé. Le résidu est partagé entre une solution aqueuse de carbonate de sodium et du dichlorométhane. La solution est extraite au dichlorométhane, lavée à l'eau, séchée ($K_2CO_3$) et évaporée pour conduire, après salification avec 2 équivalents d'éther chlorhydrique 1 N, au produit du titre sous forme d'une poudre blanche.

*Microanalyse élémentaire :*

| | C | H | N | Cl |
|---|---|---|---|---|
| % théorique | 60,30 | 7,34 | 10,55 | 17,80 |
| % expérimental | 60,16 | 7,04 | 10, 65 | 17,62 |

**EXEMPLE 2 : Chlorhydrate de *N*-(*trans*-4-{2-[(3a*S*,9b*R*)-8-cyano-1,3a,4,9b-tétrahydrochroméno[3,4-*c*]pyrrol-2 (3*H*)-yl]éthyl}cyclohexyl) acétamide**

A une solution de 699 mg (2,1 mmol) du composé de l'Exemple 1 dans 15 ml de dichlorométhane, est ajouté, à température ambiante, 354 µl de triéthylamine (1,2 eq) puis 164 µl de chlorure d'acétyle (1,1 eq). Après 2 heures d'agitation à température ambiante, le milieu est lavé à l'eau et séché ($MgSO_4$). L'évaporation du solvant suivie d'une purification sur colonne de silice avec comme éluant un mélange dichlorométhane/méthanol/ammoniaque (97 / 3 / 0,3) fournit, après salification dans l'éthanol à chaud avec de l'éther chlorhydrique 1 N, le produit du titre sous forme d'une poudre blanche.

*Point de fusion : 293 °C*

| *Microanalyse élémentaire :* | | | | |
|---|---|---|---|---|
| | *C* | *H* | *N* | *Cl* |
| *% théorique* | *65,41* | *7,49* | *10,40* | *8,78* |
| *% expérimental* | *65, 00* | *7,30* | *10,46* | *8,83* |

**EXEMPLE 3 : Chlorhydrate de *N*-(*trans*-4-{2-[(3a*S*,9b*R*)-8-cyano-1,3a,4,9b-tétrahydrochroméno[3,4-*c*]pyrrol-2(3*H*)-yl]éthy}cyclohexyl) cyclobutanecarboxamide**

Le produit du titre est obtenu selon le procédé décrit dans l'Exemple 2 en utilisant le chlorure de cyclobutanecarbonyle à la place du chlorure d'acétyle.
*Point de fusion : 297* °C

| *Microanalyse élémentaire :* | | | | |
|---|---|---|---|---|
| | *C* | *H* | *N* | *Cl* |
| *% théorique* | *67, 63* | *7,72* | *9,46* | *7,98* |
| *% expérimental* | *66,95* | *7,72* | *9, 20* | *7,90* |

**EXEMPLE 4 : Chlorhydrate de *N*-(*trans*-4-{2-[(3a*S*,9b*R*)-8-cyano-1,3a,4,9b-tétrahydrochroméno[3,4-*c*]pyrrol-2(3*H*)-yl]éthy}cyclohexyl) cyclopropanecarboxamide**

Le produit du titre est obtenu selon le procédé décrit dans l'Exemple 2 en utilisant le chlorure de cyclopropanecarbonyle à la place du chlorure d'acétyle.
*Point de fusion : 284 °C*

| *Microanalyse élémentaire :* | | | | |
|---|---|---|---|---|
| | *C* | *H* | *N* | *Cl* |
| *% théorique* | *67, 04* | *7,50* | *9,77* | *8, 25* |
| *% expérimental* | *66,32* | *7,38* | *9,49* | *8, 24* |

**EXEMPLE 5 : Chlorhydrate de *N*-(*trans*-4-(2-[(3a*S*,9b*R*)-8-cyano-1,3a,4,9b-tétrahydrochroméno[3,4-*c*]pyrrol-2(3*H*)-yl]éthyl)cyclohexyl) benzamide**

Le produit du titre est obtenu selon le protocole décrit dans l'Exemple 2 en utilisant le chlorure de benzoyle à la place du chlorure d'acétyle.
*Point de fusion : 284 °C*

| *Microanalyse élémentaire :* | | | | |
|---|---|---|---|---|
| | *C* | *H* | *N* | *Cl* |
| *% théorique* | *69,59* | *6,92* | *9,02* | *7,61* |
| *% expérimental* | *69,53* | *6,91* | *9,09* | *7,47* |

**EXEMPLE 6 : Chlorhydrate de 4-chloro-*N*-(*trans*-4-{2-[(3a*S*,9b*R*)-8-cyano-1,3a,4,9b-tétrahydrochroméno[3,4-*c*] pyrrol-2(3*H*)-yl]éthyl} cyclohexyl)benzamide**

Le produit du titre est obtenu selon le protocole décrit dans l'Exemple 2 en utilisant le chlorure de 4-chlorobenzoyle à la place du chlorure d'acétyle.
*Point de fusion : 288 °C*

| *Microanalyse élémentaire :* | | | | |
|---|---|---|---|---|
| | *C* | *H* | *N* | *Cl* |
| *% théorique* | *64,80* | *6, 24* | *8,40* | *14,17* |

(suite)

| *Microanalyse élémentaire :* | *C* | *H* | *N* | *Cl* |
|---|---|---|---|---|
| *% expérimental* | *64, 66* | *5,96* | *8,41* | *13,96* |

**EXEMPLE 7 : Chlorhydrate de *N*-(*trans*-4-{2-[(3a*S*,9b*R*)-8-cyano-1,3a,4,9b-tétrahydrochroméno[3,4-*c*]pyrrol-2(3*H*)-yl]éthyl}cyclohexyl)-2,2-diméthylpropanamide**

Le produit du titre est obtenu selon le protocole décrit dans l'Exemple 2 en utilisant le chlorure de pivaloyle à la place du chlorure d'acétyle.

*Point de fusion : 314-317 °C*

| *Microanalyse élémentaire :* | *C* | *H* | *N* | *Cl* |
|---|---|---|---|---|
| *% théorique* | *67,32* | *8,13* | *9,42* | *7,95* |
| *% expérimental* | *67,04* | *8,13* | *9,25* | *7,82* |

**EXEMPLE 8: Chlorhydrate de *N*-(*trans*-4-{2-[(3a*S*,9b*R*)-8-cyano-1,3a,4,9b-tétrahydrochroméno[3,4-*c*]pyrrol-2(3*H*)yl]éthyl}cyclohexyl) cyclopentanecarboxamide**

Le produit du titre est obtenu selon le protocole décrit dans l'Exemple 2 en utilisant le chlorure de cyclopentanecarbonyle à la place du chlorure d'acétyle.

*Point de fusion : 288 °C*

| *Microanalyse élémentaire :* | *C* | *H* | *N* | *Cl* |
|---|---|---|---|---|
| *% théorique* | *68,18* | *7,92* | *9,17* | *7,74* |
| *% expérimental* | *68,20* | *7, 69* | *9, 23* | *7,31* |

**EXEMPLE 9 : Chlorhydrate de *N*-(*trans*-4-{2-[(3a*S*,9b*R*)-8-cyano-1,3a,4,9b-tétrahydrochroméno[3,4-*c*]pyrrol-2(3*H*)-yl]éthyl}cyclohexyl)-3-pyridinesulfonamide**

Le produit du titre est obtenu selon le protocole décrit dans l'Exemple 2 en utilisant le chlorure de pyridine-3-sulfonyle (synthétisé d'après J. Org. Chem. 1989, 54, 389-393) à la place du chlorure d'acétyle.

*Point de fusion : 272 °C*

| *Microanalyse élémentaire :* | *C* | *H* | *N* | *S* | *Cl* |
|---|---|---|---|---|---|
| *% théorique* | *59,69* | *6,21* | *11,14* | *6,37* | *7,05* |
| *% expérimental* | *59,06* | *6, 02* | *10,93* | *5,48* | *7, 06* |

**EXEMPLE 10 : Chlorhydrate de *N*-(*trans*-4-{2-[(3a*S*,9b*R*)-8-cyano-1,3a,4,9b-tétrahydrochroméno[3,4-*c*]pyrrol-2(3*H*)-yl]éthyl}cyclohexyl)-4-fluorobenzènesulfonamide**

Le produit du titre est obtenu selon le protocole décrit dans l'Exemple 2 en utilisant le chlorure de 4-fluorobenzènesulfonyle à la place du chlorure d'acétyle.

*Point de fusion : 300 °C*

| *Microanalsys élémentaire :* | *C* | *H* | *N* | *S* | *Cl* |
|---|---|---|---|---|---|
| *% théorique* | *60,05* | *6,01* | *8,08* | *6,17* | *6,82* |
| *% expérimental* | *59,28* | *5,90* | *7,90* | *5, 68* | *6,49* |

**<u>EXEMPLE 11</u> : Chlorhydrate de 4-chloro-*N*-(*trans*-4-{2-[(3a*S*,9b*R*)-8-cyano-1,3a,4,9b-tétrahydrochroméno [3,4-*c*]pyrrol-2(3*H*)-yl]éthyl} cyclohexyl)benzènesulfonamide**

Le produit du titre est obtenu selon le protocole décrit dans l'Exemple 2 en utilisant le chlorure de 4-chlorobenzènesulfonyle à la place du chlorure d'acétyle.
*<u>Point de fusion</u> : 285 °C*

*<u>Microanalyse élémentaire :</u>*

| | *C* | *H* | *N* | *S* | *Cl* |
|---|---|---|---|---|---|
| *% théorique* | *58,21* | *5,82* | *7,83* | *5,98* | *13,22* |
| *% expérimental* | *58,53* | *5,70* | *7,70* | *5,68* | *12,75* |

**<u>EXEMPLE 12</u> : Chlorhydrate de l'acide 4-[(*trans*-4-{2-[(3a*S*,9b*R*)-8-cyano-1,3a,4,9b-tétrahydrochroméno[3,4-*c*] pyrrol-2(3*H*)-yl]éthyl}cyclohexyl) carbamoyl]benzoïque**

*<u>Stade A</u> : 4-[(trans-4-{2-[(3a*S, *9b*R*)-8-cyano-1,3a,4,9b-tétrahydrochroméno [3,4-*c*]pyrrol-2(3*H*)-yl]ethyl}cyclohexyl) carbamoyl]benzoate de méthyle*

Le produit du titre est obtenu selon le protocole décrit dans l'Exemple 2 en utilisant le 4-chlorocarbamoylbenzoate de méthyle à la place du chlorure d'acétyle.

*<u>Stade B</u>: Chlorhydrate de l'acide 4-[(*trans*-4-{2-[(3a*S*,96*R*)-8-cyano-7,3a,4,9b-tétrahydrochroméno[3,4-*c*]pyrrol-2 (3*H*)-yl]éthyl}cyclohexyl) carbamoyl]benzoïque*

0.737 g du produit obtenu au Stade précédent (1,68 mmol) est solubilisé dans 15 ml d'éthanol et porté au reflux avec 1,79 ml d'une solution de soude 1 N pendant 1,5 heures. L'éthanol est alors évaporé, le milieu réactionnel repris dans de l'eau puis 3,6 ml d'acide chlorhydrique 1 N est ajouté à 0 °C. Le solide obtenu est filtré et séché au dessiccateur.
*<u>Point de fusion</u> : 298 °C*

*<u>Microanalyse élémentaire :</u>*

| | *C* | *H* | *N* | *Cl* |
|---|---|---|---|---|
| *% théorique* | *65,94* | *6,32* | *8, 24* | *6,95* |
| *% expérimental* | *66,22* | *5,83* | *8,19* | *6,28* |

**<u>EXEMPLE 13</u> : Chlorhydrate de l'acide 2-[(*trans*-4-{2-[(3a*S*,9b*R*)-8-cyano-1,3a,4,9b-tétrahydrochroméno[3,4-*c*] pyrrol-2(3*H*)-yl]éthyl}cyclohexyl) carbamoyl]benzoïque**

*<u>Stade A</u> : 4-[(*trans*-4-{2-[(3a*S*,9b*R*)-8-cyano-1,3a,4,9b-tétrahydrochroméno [3,4-*c*]pyrrol-2(3*H*)-yl]ethyl}cyclohexyl) carbamoyl]benzoate de méthyle*

Une solution de 1,21 g du composé de l'Exemple 1 (sous forme base) (3,72 mmol), de 0,8 g du composé de la Préparation 7 (1,2eq), de 0,78 g de chlorhydrate de 1-(3-diméthylaminopropyl)-3-éthyl-carbodümide (1,1 eq), et de 50 mg d'hydroxy-benzotriazole dans 80 ml de dichlorométhane est agitée 18 heures à température ambiante. 37 ml d'une solution aqueuse saturée en bicarbonate de sodium est ajoutée puis le mélange est agité 15 minutes. Après extraction au dichlorométhane, séchage ($MgSO_4$), évaporation, le résidu est purifié par chromatographie sur colonne de silice avec comme éluant un mélange dichlorométhane/méthanol/ammoniaque (97/3/0,3) pour conduire au produit du titre sous forme d'une huile.

*<u>Stade B</u> : Chlorhydrate de l'acide 2-[(trans-4-{2-[(3aS,9bR)-8-cyano-1,3a,4,9b-tétrahydrochroméno[3,4-c]pyrrol-2 (3H)-yl]éthyl}cyclohexyl) carbamoyl]benzoïque*

Le produit du titre est obtenu selon le procédé décrit le Stade B de l'Exemple 12, en prenant comme produit de départ le composé obtenu dans le Stade A précédent.
*<u>Point de fusion</u> : 303 °C*

| *Microanalyse élémentaire :* | | | | |
|---|---|---|---|---|
| | *C* | *H* | *N* | *Cl* |
| *% théorique* | *65,94* | *6,32* | *8,24* | *6,95* |
| *% expérimental* | *65,57* | *6, 25* | *8,15* | *6,52* |

**EXEMPLE 14 : Chlorhydrate de l'acide 3-[(*trans*-4-{2-[(3a*S*,9b*R*)-8-cyano-1,3a,4,9b-tétrahydrochroméno[3,4-*c*] pyrrol-2(3*H*)-yl]éthyl}cycloheayl) carbamoyl]benzoïque**

Le produit du titre est obtenu selon le procédé décrit dans l'Exemple 13 en utilisant l'isophtalate de monométhyle commercial à la place de la Préparation 7.

*Point de fusion* : 257 *°C*

| *Microanalyse élémentaire :* | | | | |
|---|---|---|---|---|
| | *C* | *H* | *N* | *Cl* |
| *% théorique* | *65,94* | *6,32* | *8,24* | *6,95* |
| *% expérimental* | *65,33* | *6,15* | *8,03* | *6,55* |

**EXEMPLE 15 : Dichlorhydrate de *N*-(*trans*-4-{2-[(3a*S*,9b*R*)-8-cyano-1,3a,4,9b-tétrahydrochroméno[3,4-*c*]pyrrol-2(3*H*)-yl]éthyl}cyclohexyl) azétidine-3-carboxamide**

*Stade A : 3-[(*trans*-4-{2-[(3a*S*,9b*R*)-8-cyano-1,3a,4,9b-tétrahydrochroméno [3,4-*c*]pyrrol-2(3*H*)-yl]éthyl}cyclohexyl) carbamoyl]azétidine-1-carboxylate de* tert*-butyle*

Le produit du titre est obtenu selon le procédé décrit dans le Stade A de l'Exemple 13 en utilisant l'acide 1-(tert-butoxycarbonyl)azétidine-3-carboxylique (synthétisé d'après Biochemistry, 2001, 40, 5226-5232) à la place de la Préparation 7.

*Stade B : Dichlorhydrate de* N-(trans*-4-{2-[(3a*S*,9b*R*)-8-cyano-1,3a,4,9b-tétrahydrochroméno[3,4-*c*]pyrrol-2(3*H*)-yl] éthyl)cyclohexyl)azétidine-3-carboxamide*

2,82 g du produit obtenu au Stade précédent (5,56 mmol) est agité à température ambiante dans un mélange 56 ml de dichlorométhane et 5,6 ml acide trifluoroacétique pendant 3 heures. Les solvants sont alors évaporés, de la soude concentrée est ensuite ajoutée et la solution est ensuite extraite à l'acétate d'éthyle, lavée avec une solution de soude 1 N, avec de la saumure, séchée ($MgSO_4$) et évaporée. Après salification dans l'éthanol à chaud avec de l'éther chlorhydrique 1 N, le produit du titre est obtenu sous la forme d'une poudre.

*Point de fusion: 283 °C*

| *Microanalyse élémentaire :* | | | | |
|---|---|---|---|---|
| | C | *H* | *N* | *Cl* |
| *% théorique* | *59,87* | *7,12* | *11,64* | *14,73* |
| *% expérimental* | *59,67* | *6,84* | *11,42* | *14,56* |

**EXEMPLE 16 : Chlorhydrate de 1-acétyl-*N*-(*trans*-4-{2-[(3a*S*,9b*R*)-8-cyano-1,3a,4,9b-tétrahydrochroméno [3,4-*c*]pyrrol-2(3*H*)-yl]éthyl} cyclohexyl)azétidine-3-carboxamide**

Le produit du titre est obtenu selon le procédé décrit dans l'Exemple 2 en utilisant comme produit de départ le composé de l'Exemple 15 à la place du composé de l'Exemple 1.

*Point de fusion* : 251 *°C*

| *Microanalyse élémentaire:* | | | | |
|---|---|---|---|---|
| | *C* | *H* | *N* | *Cl* |
| *% théorique* | *64,12* | *7, 24* | *11,50* | *7, 28* |

(suite)

| *Microanalyse élémentaire:* | | | | |
|---|---|---|---|---|
| | *C* | *H* | *N* | *Cl* |
| *% expérimental* | *63,88* | *7,16* | *11,29* | *7, 27* |

**EXEMPLE 17 : Chlorhydrate de *N*-(*trans*-4-{2-[(3a*S*,9b*R*)-8-cyano-1,3a,4,9b-tétrahydrochroméno[3,4-*c*]pyrrol-2(3*H*)-yl]éthyl}cyclohexyl)-3,3-difluorocyclobutanecarboxamide**

Le produit du titre est obtenu selon le procédé décrit dans le Stade A de l'Exemple 13 en utilisant l'acide 3,3-difluorocyclobutanecarboxylique (synthétisé selon Synthetic Communications, 2005, 35, 657-662) à la place de la Préparation 7.

Point de fusion : 288 °C

| *Microanalyse élémentaire :* | | | | |
|---|---|---|---|---|
| | *C* | *H* | *N* | *Cl* |
| *% théorique* | *62,56* | *6,72* | *8,75* | *7,39* |
| *% expérimental* | *63,17* | *6, 60* | *8,73* | *7,35* |

**EXEMPLE 18 : Chlorhydrate de *cis-N*-(*trans*-4-{2-[(3a*S*,9b*R*)-8-cyano-1,3a,4,9b-tétrahydrochroméno[3,4-*c*]pyrrol-2(3*H*)-yl]éthyl}cyclohexyl)-3-hydroxycyclobutanecarboxamide**

Le produit du titre est obtenu selon le procédé décrit dans le Stade A de l'Exemple 13 en utilisant l'acide 3-*cis*-hydroxycyclobutanecarboxylique (synthétise selon la demande de brevet US 2005/0020645) à la place de la Préparation 7.

Point de fusion : *269 °C*

| *Microanalyse élémentaire :* | | | | |
|---|---|---|---|---|
| | *C* | *H* | *N* | *Cl* |
| *% théorique* | *65,28* | *7,45* | *9,13* | *7,71* |
| *% expérimental* | *65,28* | *7, 26* | *9,07* | *7,65* |

**EXEMPLE 19 : Chlorhydrate de (3a*S*,9b*R*)-2-{2-[*trans*-4-(2-oxopyrrolidin-1-yl) cyclohexyl]éthyl}-1,2,3,3a,4,9b-hexahydrochroméno[3,4-*c*]pyrrole-8-carbonitrile**

*Stade A : 4-chloro-*N-*(*trans*-4-{2-[(3a*S*,9b*R*)-8-cyano-1,3a,4,9b-tétrahydro chroméno[3,4-*c*]pyrrol-2(3*H*)-yl]éthyl}cyclohexyl)butanamide*

Le produit du titre est obtenu selon le procédé décrit dans l'Exemple 2 en utilisant le chlorure de 4-chlorobutyryle à la place du chlorure d'acétyle.

*Stade B : Chlorhydrate de (3a*S*,9b*R*)-2-{2-[*trans*-4-(2-oxopyrrolidin-1-yl) cyclohexyl]éthyl}-1,2,3,3a,4,9b-hexahydrochroméno[3,4-*c*]pyrrole-8-carbonitrile*

1,77 g du produit obtenu au Stade précédent (4,12 mmol) est mis en suspension dans 3 ml de THF avec 165 mg d'hydrure de sodium, puis chauffé au reflux 18 heures. Le mélange refroidi est alors versé sur une solution aqueuse saturée en chlorure d'ammonium. Le précipité obtenu est purifié par une chromatographie sur colonne de silice avec comme éluant un mélange dichlorométhane/méthanol/ammoniaque (98 / 2 / 0,2) pour conduire, après salification dans l'éthanol à chaud avec de l'éther chlorhydrique 1 N, au produit du titre.

Point de fusion : *260-262 °C*

**EXEMPLE 20 : 2-[(*trans*-4-{2-[(3a*S*,9b*R*)-8-cyano-1,3a,4,9b-tétrahydrochroméno [3,4-*c*]pyrrol-2(3*H*)-yl]éthyl}cyclohexyl)amino]-3,4-dioxocyclobutèn-1-olate de sodium**

*Stade A : (3a*S*, 9b*R*)-2-(2-{*trans*-4-[(2-éthoxy-3,4-dioxocyclobutèn-1-yl)amino] cyclohexyl}éthyl)-1,2,3,3a,4,9b-hexahydrochroméno[3,4-*c*]pyrrole-8-carbonitrile*

2 g du composé de l'Exemple 1 (6,15 mmol) et 0,9 ml de squarate de diéthyle sont agités à température ambiante pendant 2 heures dans 15 ml d'éthanol. Après ajout d'eau, les cristaux obtenus sont filtrés et séchés au dessiccateur pour conduire au produit du titre sous forme d'une poudre blanche.

*Stade B : 2-[(*trans*-4-{2-[(3a*S*,9b*R*)-8-cyano-1,3a,4,9b-tétrahydrochroméno [3,4-*c*]pyrrol-2(3*H*)yl]éthyl}cyclohexyl)amino]-3,4-dioxocyclobutèn-1-olate de sodium*

1,3 g du produit obtenu au Stade précédent est mis en suspension dans une solution de 29 ml d'eau et 6 ml d'éthanol avec 289 mg de pastilles de soude à 90 °C. On rajoute à la solution refroidie de l'acétone et filtre le précipité obtenu correspondant au produit du titre.
*Spectrométrie de masse : $[M+H]^+$ = 421*

**EXEMPLE 21 : Chlorhydrate de (3a*S*,9b*R*)-2-{2-[*trans*-4-[(2-amino-3,4-dioxo cyclobutèn-1-yl)amino]cyclohexyl] éthyl}-1,2,3,3a,4,9b-hexahydro chroméno[3,4-*c*]pyrrole-8-carbonitrile**

Une solution de 1,17 g du composé obtenu dans le Stade A de l'Exemple 20 (2,61 mmol) dans 13 ml d'éthanol est traitée avec 13 ml d'une solution saturée d'ammoniac dans l'acétonitrile pendant 16 heures. Le solide obtenu est filtré puis lavé à l'acétate d'éthyle pour enfin conduire, après salification dans l'éthanol à chaud avec de l'éther chlorhydrique 1 N, au produit du titre.
*Point de fusion : 325 °C*

*Microanalyse élémentaire :*

| | C | H | N | Cl |
|---|---|---|---|---|
| % théorique | 63,08 | 6,40 | 12,26 | 7,76 |
| % expérimental | 62,60 | 6, 28 | 11,95 | 7,76 |

**EXEMPLE 22 : Dichlorhydrate de (3a*S*,9b*R*)-2-{2-[*trans*-4-(méthylamino) cyclohexyl]éthyl}-1,2,3,3a,4,9b-hexahydrochroméno[3,4-*c*]pyrrole-8-carbonitrile**

Le produit du titre est obtenu selon le procédé décrit dans l'Exemple 1 en prenant comme produit de départ le composé obtenu dans la Préparation 4.
*Point de fusion : 320 °C*

*Microanalyse élémentaire :*

| | C | H | N | Cl |
|---|---|---|---|---|
| % théorique | 61,16 | 7,58 | 10,19 | 17,19 |
| % expérimental | 60,96 | 7,30 | 10,09 | 17,28 |

**EXEMPLE 23 : Chlorhydrate de *N*-(*trans*-4-{2-[(3a*S*,9b*R*)-8-cyano-1,3a,4,9b-tétrahydrochroméno[3,4-*c*]pyrrol-2(3*H*)-yl]éthyl}cyclohexyl)-*N*-méthylacétamide**

Le produit du titre est obtenu selon le procédé décrit dans l'Exemple 2 en prenant comme produit de départ le composé de l'Exemple 22 à la place du composé de l'Exemple 1.
*Point de fusion : 248 °C*

*Microanalyse élémentaire :*

| | C | H | N | Cl |
|---|---|---|---|---|
| % théorique | 66,09 | 7, 72 | 10,05 | 8,48 |
| % expérimental | 65,06 | 7,18 | 9,82 | 8,89 |

**EXEMPLE 24 : Chlorhydrate de *N*-(trans-4-{2-[(3a*S*,9b*R*)-8-cyano-1,3a,4,9b-tétrahydrochroméno[3,4-*c*]pyrrol-2(3*H*)-yl]éthyl}cyclohexyl)-*N*-méthylcyclobutanecarboxamide**

Le produit du titre est obtenu selon le procédé décrit dans l'Exemple 3 en prenant comme produit de départ le composé de l'Exemple 22 à la place du composé de l'Exemple 1.

*Point de fusion : 244 °C*

*Microanalyse élémentaire :*

| | C | H | N | Cl |
|---|---|---|---|---|
| % théorique | 68,18 | 7,92 | 9,17 | 7,74 |
| % expérimental | 67,55 | 7, 23 | 9, 06 | 7, 69 |

**EXEMPLE 25 : Dichlorhydrate de (3a*S*,9b*R*)-2-{2-[*trans*-4-(diméthylamino) cyclohexyl]éthyl}-1,2,3,3a,4,9b-hexahydrochroméno[3,4-*c*]pyrrole-8-carbonitrile**

A une solution de 0,685 g du composé de l'Exemple 1 (1,95 mmol) dans 18 ml de méthanol, sont ajoutés à 0 °C 0,245 g de cyanoborohydrure de sodium, 0,56 ml d'acide acétique puis, goutte à goutte, 0,4 ml d'une solution de formaldéhyde 37 % dans l'eau. Après 4 heures d'agitation, 2 ml d'une solution aqueuse saturée en carbonate de potassium est ajouté. Après évaporation des solvants puis dilution dans de l'eau, le milieu est extrait à l'acétate d'éthyle, séché ($MgSO_4$) et évaporé. Le résidu obtenu est purifié par chromatographie sur colonne de silice avec comme éluant un mélange dichlorométhane/méthanol/ammoniaque (80 / 20 / 2) pour conduire, après salification dans l'éthanol à chaud avec de l'éther chlorhydrique 1 N, au produit du titre.

*Point de fusion : 305 °C*

*Microanalyse élémentaire :*

| | C | H | N | Cl |
|---|---|---|---|---|
| % théorique | 61,97 | 7,80 | 9,85 | 16,63 |
| % expérimental | 61,98 | 7,52 | 9,43 | 16,42 |

**EXEMPLE 26 : Dichlorhydrate de (3a*S*,9b*R*)-2-{2-[*trans*-4-(pyridin-2-ylamino) cyclohexyl]éthyl}-1,2,3,3a,4,9b-hexahydrochroméno[3,4-*c*]pyrrole-8-carbonitrile**

Un mélange de 0,482 g du composé de l'Exemple 1 (1,48 mmol) et de 0,245 ml de diisopropyléthylamine dans 5 ml de 2-fluoropyridine est porté au reflux 24 heures. Après évaporation des solvants puis dilution dans de l'eau, le milieu est extrait à l'acétate d'éthyle, séché ($MgSO_4$) et évaporé. Le résidu obtenu est purifié par chromatographie sur colonne de silice avec comme éluant un mélange dichlorométhane/méthanol/ammoniaque (97 / 3 / 0,3) pour conduire, après salification dans l'éthanol à chaud avec de l'éther chlorhydrique 1 N, au produit du titre.

*Point de fusion :* 305 °C

*Microanalyse élémentaire :*

| | C | H | N | Cl |
|---|---|---|---|---|
| % théorique | 63,15 | 6,78 | 11,78 | 14,91 |
| % expérimental | 63,11 | 6,57 | 11,09 | 14,52 |

**EXEMPLE 27 : Dichlorhydrate de (3a*S*,9b*R*)-2-{2-[*trans*-4-(pyrimidin-2-ylamino) cyclohexyl]éthyl}-1,2,3,3a,4,9b-hexahydrochroméno[3,4-*c*]pyrrole-8-carbonitrile**

Une suspension de 0,715 g du composé de l'Exemple 1 (2,2 mmol), de 0,303 mg de 2-chloropyrimidine et de 0,47 g de carbonate de sodium dans 45 ml d'éthanol est portée au reflux 72 heures. Après filtration, les solvants sont alors évaporés. Après extraction à l'acétate d'éthyle, séchage ($MgSO_4$), l'évaporation fournit une huile purifiée par chromatographie sur colonne de silice avec comme éluant un mélange dichlorométhane/méthanol/ammoniaque (98 / 2 / 0,2) pour conduire, après salification dans l'éthanol à chaud avec de l'éther chlorhydrique 1 N, au produit du titre.

*Point de fusion : 234 °C*

*Microanalyse élémentaire :*

| | *C* | *H* | *N* | *Cl* |
|---|---|---|---|---|
| *% théorique* | *60,50* | *6,56* | *14,70* | *14,88* |
| *% expérimental* | *60,75* | *6, 26* | *14,58* | *14,24* |

**ETUDE PHARMACOLOGIQUE**

**<u>EXEMPLE A</u> : Détermination de l'affinité pour les récepteurs $D_3$, $D_{2S}$ et $D_{2L}$ humains exprimés de manière stable en cellules CHO.**

L'affinité des composés pour les récepteurs dopaminergiques $D_3$, $D_{2S}$ et $D_{2L}$ humains est évaluée par des expériences de compétition avec la spipérone tritiée ([$^3$H]-spipérone) sur des membranes de cellules CHO exprimant ces 3 sous-types de récepteurs de manière stable. Les différentes lignées cellulaires sont mises en culture jusqu'à confluence puis les cellules sont décollées et homogénéisées au Polytron dans un tampon de Tris-HCl (50 mM, pH 7,4) contenant 5 mM de $MgCl_2$. Le broyat cellulaire est ensuite centrifugé (15 minutes à 4 °C) et le culot est repris dans un volume approprié de tampon HEPES (20 mM) contenant 150 mM de NaCl et congelé à -80 °C. Le jour de l'expérience, les membranes sont reprises dans le tampon d'incubation contenant 50 mM de Tris (pH 7,4), 120 mM de NaCl, 5 mM de KCl, 2 mM de $CaCl_2$ et 5 mM de $MgCl_2$. Les membranes sont ensuite incubées pendant 1 heure à 30 °C avec le composé étudié en présence de 0,5 nM de [$^3$H]-spipérone. La liaison non-spécifique est déterminée avec 10 μM de raclopride. A la fin de la période d'incubation, les échantillons sont filtrés au travers de filtres de type Unifilter GF/B pré-traités au PEI (0,1 %) et lavés plusieurs fois avec le tampon de filtration Tris-HCl (50 mM, pH 7,4) contenant 120 mM de NaCl. La radioactivité retenue sur les filtres est comptée après adjonction de liquide scintillant à l'aide d'un compteur à scintillation. Les isothermes obtenues sont analysés par régression non-linéaire afin de déterminer des valeurs d'$IC_{50}$ qui sont converties en $pK_i$ selon l'équation de Cheng-Prussof :

$$K_i = IC_{50}/(1 + L/K_d)$$

dans laquelle L représente la concentration en radioligand et le $K_d$ est la constante de dissociation de la [$^3$H]-spipérone sur les récepteurs dopaminergiques $D_3$, $D_{2S}$ et $D_{2L}$ (0,26, 0,14 et 0,156 et, respectivement). Les résultats sont exprimés en $pK_i = - \log K_i$.

| | Affinité ($pK_i$) | | |
|---|---|---|---|
| | $hD_3$ | $hD_{2L}$ | $hD_{2S}$ |
| **Exemple 2** | 8,70 ± 0,05 | 7,0 ± 0,04 | 6,93 ± 0,04 |
| **Exemple 3** | 8,73 ± 0,11 | 7,30 ± 0,05 | 7,25 ± 0,11 |

**<u>EXEMPLE B</u> : Inhibition de l'induction de l'hypothermie induite par l'agoniste dopaminergique PD128,907.**

Sur des rats mâles de souche Wistar, la température basale (rectale) est mesurée puis le composé à tester ou le véhicule est administré par voie orale. Soixante minutes plus tard, le PD128,907 (0,63 mg/kg, s.c), un agoniste dopaminergique $D_3/D_2$, est injecté. Trente minutes plus tard, la température corporelle est re-déterminée et la différence (Δ) avec la température basale est calculée.

| Traitement T-60 min | Dose (mg/kg, p.o.) | Traitement T0 | Dose (mg/kg, s.c.) | Δ (°C) ± S.E.M.s | $DI_{50}$ (mg/kg, p.o.) |
|---|---|---|---|---|---|
| *Véhicule* | *0* | *Véhicule* | *0* | *+0,36 ± 0,12* | |
| *Véhicule* | *0* | *PD128,907* | *0,63* | *-1,63 ± 0,25§* | |
| **Exemple 2** | 0,04 | PD128,907 | 0,63 | -1,73 ± 0,26 | |
| | 0,16 | PD128,907 | 0,63 | -1,33 ± 0,11 | **1,33** |
| | 0,63 | PD128,907 | 0,63 | -0,97 ± 0,28 | |

(suite)

| Traitement T-60 min | Dose (mg/kg, p.o.) | Traitement T0 | Dose (mg/kg, s.c.) | Δ (°C) ± S.E.M.s | $DI_{50}$ (mg/kg, p.o.) |
|---|---|---|---|---|---|
| | 2,5 | PD128,907 | 0,63 | -0,4 ± 0,13* | |
| | 10,0 | PD128,907 | 0,63 | +0,1 ± 0,33* | |
| *Véhicule* | *0* | *Véhicule* | *0* | *+0,68 ± 0,10* | |
| *Véhicule* | *0* | *PD128, 907* | *0, 63* | *-1,60 ± 0,22§* | |
| **Exemple 3** | 0,04 | PD128,907 | 0,63 | -1,65 ± 0,09 | |
| | 0,16 | PD128,907 | 0,63 | -1,62 ± 0,07 | **0,64** |
| | 0,63 | PD128,907 | 0,63 | -0,83 ± 0,20* | |
| | 2,5 | PD128,907 | 0,63 | -0,33 ± 0,16* | |
| | 10,0 | PD128,907 | 0,63 | + 0,35 ± 0,18 * | |

$DI_{50}$ = Dose Inhibitrice$_{50}$
§ Différence significative versus "*Véhicule + Véhicule*" (test t de Student, P < 0,05)
* Différence significative versus "*Véhicule + PD128,907*" (test de Dunnett après ANOVA, P < 0,05).

En présence du véhicule, le PD128,907 provoque une hypothermie qui est bloquée de façon dose-dépendante par le composé de l'Exemple 3 démontrant une activité antagoniste des récepteurs $D_3/D_2$.

**EXEMPLE C : Composition pharmaceutique**

| | |
|---|---|
| Formule de préparation pour 1000 comprimés dosés à 10 mg en chlorhydrate de *N*-(*trans*-4-{2-[(3a*S*,9b*R*)-8-cyano-1,3a,4,9b-tétrahydrochroméno[3,4-*c*]pyrrol-2(3*H*)-yl] éthyl}cyclohexyl)cyclobutanecarboxamide (Exemple 3) | 10g |
| Hydroxypropylcellulose | 2 g |
| Amidon de blé | 10 g |
| Lactose | 100 g |
| Stéarate de magnésium | 3 g |
| Talc | 3 g |

## Revendications

**1.** Composés de formule (I) :

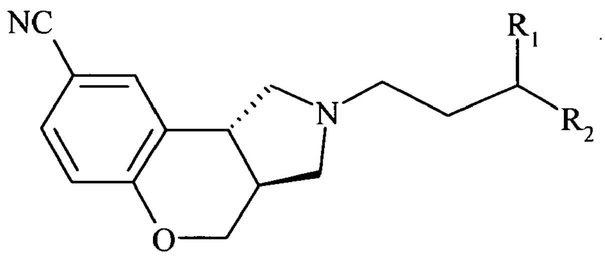

(I)

dans laquelle $R_1$ et $R_2$ forment ensemble la chaîne carbonée suivante :

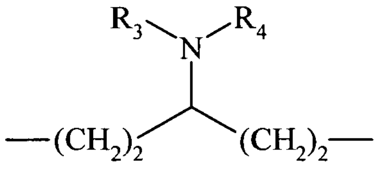

dans laquelle :

➢ $R_3$ représente un atome d'hydrogène ou un groupement alkyle ($C_1$-$C_6$) linéaire ou ramifié,
➢ $R_4$ représente :

- un atome d'hydrogène,
- un groupement alkyle ($C_1$-$C_6$) linéaire ou ramifié, aryle, hétéroaryle ou 3,4-dioxocyclobutenyle, chacun de ces groupements étant éventuellement substitué par un ou plusieurs groupements, identiques ou différents, choisis parmi halogène ; alkyle ($C_1$-$C_6$) linéaire ou ramifié ; alkylcarbonyle ($C_1$-$C_6$) linéaire ou ramifié ; carboxy ; hydroxy ; cyano ; nitro ; aminocarbonyle non-substitué ou substitué par un ou plusieurs groupements alkyle ($C_1$-$C_6$) linéaire ou ramifié ; amino non-substitué ou substitué par un ou deux groupements alkyle ($C_1$-$C_6$) linéaire ou ramifié,
- un groupement $-COR_5$,
- un groupement $-SO_2R_5$,

➢ $R_5$ représente un groupement alkyle ($C_1$-$C_6$) linéaire ou ramifié, cycloalkyle ($C_3$-$C_8$), hétérocycloalkyle, aryle ou hétéroaryle, chacun de ces groupements étant éventuellement substitué par un ou plusieurs groupements, identiques ou différents, choisis parmi halogène ; alkyle ($C_1$-$C_6$) linéaire ou ramifié ; alkylcarbonyle ($C_1$-$C_6$) linéaire ou ramifié ; carboxy ; hydroxy ; cyano ; nitro ; aminocarbonyle non-substitué ou substitué par un ou plusieurs groupements alkyle ($C_1$-$C_6$) linéaire ou ramifié ; amino non-substitué ou substitué par un ou deux groupements alkyle ($C_1$-$C_6$) linéaire ou ramifié,
➢ ou bien, $R_3$ et $R_4$ forment ensemble avec l'atome d'azote qui les porte un cycle comportant de 5 à 8 chaînons, le cycle ainsi défini pouvant être éventuellement substitué par un ou plusieurs groupements, identiques ou différents, choisis parmi halogène ; alkyle ($C_1$-$C_6$) linéaire ou ramifié ; hydroxy ; oxo ; amino non-substitué ou substitué par un ou deux groupements alkyle ($C_1$-$C_6$) linéaire ou ramifié,
leurs isomères de position, leurs énantiomères, leurs diastéréoisomères, ainsi que leurs sels d'addition à un acide ou à une base pharmaceutiquement acceptable.

2. Composés de formule (I) selon la revendication 1, **caractérisés en ce que** le groupement $R_3$ représente un atome d'hydrogène ou un groupement méthyle.

3. Composés de formule (I) selon la revendication 1 ou 2, **caractérisés en ce que** le groupement $R_4$ représente un atome d'hydrogène ou un groupement alkyle ($C_1$-$C_6$) linéaire ou ramifié.

4. Composés de formule (I) selon la revendication 1 ou 2, **caractérisés en ce que** le groupement $R_4$ représente un groupement $-COR_5$, où $R_5$ est tel que défini dans la revendication 1.

5. Composés de formule (I) selon la revendication 1 ou 2, **caractérisés en ce que** le groupement $R_4$ représente un groupement $-SO_2R_5$, où $R_5$ est tel que défini dans la revendication 1.

6. Composés de formule (I) selon la revendication 1, 2, 4 ou 5, **caractérisés en ce que** le groupement $R_5$ représente un groupement alkyle ($C_1$-$C_6$) linéaire ou ramifié.

7. Composés de formule (I) selon la revendication 1, 2, 4 ou 5, **caractérisés en ce que** le groupement $R_5$ représente un groupement cycloalkyle ($C_3$-$C_8$), éventuellement substitué.

8. Composés de formule (I) selon la revendication 1, 2, 4 ou 5, **caractérisés en ce que** le groupement $R_5$ représente un groupement aryle, éventuellement substitué.

9. Composés de formule (I) selon la revendication 1, 2, 4 ou 5, **caractérisés en ce que** le groupement $R_5$ représente

un groupement hétéroaryle, éventuellement substitué.

**10.** Composés de formule (I) selon la revendication 1, **caractérisés en ce que** $R_3$ et $R_4$ forment ensemble avec l'atome d'azote qui les porte un cycle à 5 chaînons, le cycle ainsi formé pouvant être éventuellement substitué.

**11.** Composés de formule (I) selon la revendication 1 qui sont le :

- (3a*S*,9b*R*)-2-[2-(*trans*-4-aminocyclohexyl)éthyl]-1,2,3,3a,4,9b-hexahydrochroméno [3,4-c]pyrrole-8-carbonitrile ;
- (3a*S*,9b*R*)-2-{2-[*trans*-4-(méthylamino)cyclohexyl]éthyl}-1,2,3,3a,4,9b-hexahydro chroméno[3,4-c]pyrrole-8-carbonitrile ;
- (3a*S*,9b*R*)-2-{2-[*trans*-4-(diméthylamino)cyclohexyl]éthyl}-1,2,3,3a,4,9b-hexahydrochroméno[3,4-*c*]pyrrole-8-carbonitrile.
- *N*-(*trans*-4-{2-[(3a*S*,9b*R*)-8-cyano-1,3a,4,9b-tétrahydrochroméno[3,4-*c*]pyrrol-2(3*H*)-yl]éthyl}cyclohexyl) acétamide ;
- *N*-(*trans*-4-{2-[(3a*S*,9b*R*)-8-cyano-1,3a,4,9b-tétrahydrochroméno[3,4-*c*]pyrrol-2(3*H*)-yl]éthyl}cyclohexyl)-2,2-diméthylpropanamide ;
- *N*-(*trans*-4-{2-[(3a*S*,9b*R*)-8-cyano-1,3a,4,9b-tétrahydrochroméno[3,4-*c*]pyrrol-2(3*H*)-yl]éthyl}cyclohexyl)-*N*-méthylacétamide.
- *N*-(*trans*-4-{2-[(3a*S*,9b*R*)-8-cyano-1,3a,4,9b-tétrahydrochroméno[3,4-*c*]pyrrol-2(3*H*)-yl]éthyl}cyclohexyl) cyclobutanecarboxamide ;
- *N*-(*trans*-4-{2-[(3a*S*,9b*R*)-8-cyano-1,3a,4,9b-tétrahydrochroméno[3,4-c]pyrrol-2(3*H*)-yl]éthyl}cyclohexyl) cyclopropanecarboxamide ;
- *N*-(*trans*-4-{2-[(3a*S*,9b*R*)-8-cyano-1,3a,4,9b-tétrahydrochroméno[3,4-*c*]pyrrol-2(3*H*)-yl]éthyl}cyclohexyl)-3,3-difluorocyclobutanecarboxamide;
- *cis*-*N*-(*trans*-4-{2-[(3a*S*,9b*R*)-8-cyano-l,3a,4,9b-tétrahydrochroméno[3,4-*c*]pyrrol-2(3*H*)-yl]éthyl}cyclohexyl)-3-hydroxycyclobutanecarboxamide,
- *N*-(*trans*-4-{2-[(3a*S*,9b*R*)-8-cyano-1,3a,4,9b-tétrahydrochroméno[3,4-*c*]pyrrol-2(3*H*)-yl]éthyl}cyclohexyl)-*N*-méthylcyclobutanecarboxamide.
- *N*-(*trans*-4-{2-[(3a*S*,9b*R*)-8-cyano-1,3a,4,9b-tétrahydrochroméno[3,4-*c*]pyrrol-2(3*H*)-yl]éthyl}cyclohexyl) benzamide ;
- 4-chloro-*N*-(*trans*-4-{2-[(3a*S*,9b*R*)-8-cyano-1,3a,4,9b-tétrahydrochroméno[3,4-*c*] pyrrol-2(3*H*)-yl]éthyl}cyclohexyl)benzamide.
- *N*-(*trans*-4-{2-[(3a*S*,9b*R*)-8-cyano-1,3a,4,9b-tétrahydrochroméno[3,4-*c*]pyrrol-2(3*H*)-yl]éthyl}cyclohexyl)-4-fluorobenzènesulfonamide ;
- 4-chloro-*N*-(*trans*-4-{2-[(3a*S*,9b*R*)-8-cyano-1,3a,4,9b-tétrahydrochroméno [3,4-*c*]pyrrol-2(3*H*)-yl]éthyl}cyclohexyl)benzènesulfonamide ;
- *N*-(*trans*-4-{2-[(3a*S*,9b*R*)-8-cyano-1,3a,4,9b-tétrahydrochroméno[3,4-*c*]pyrrol-2(3*H*)-yl]éthyl}cyclohexyl)-3-pyridinesulfonamide.
- (3a*S*,9b*R*)-2-{2-[*trans*-4-(2-oxopyrrolidin-1-yl)cyclohexyl]éthyl}-1,2,3,3a,4,9b-hexahydrochroméno[3,4-*c*] pyrrole-8-carbonitrile.

**12.** Procédé de préparation des composés de formule (I) selon la revendication 1, **caractérisé en ce que** l'on utilise comme produit de départ un composé de formule (II) :

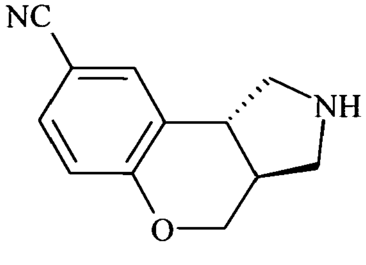

(II)

qui subit une réaction d'amination réductrice en présence d'un agent réducteur, tel que le triacétoxyborohydrure ou le cyanoborohydrure de sodium, et d'un composé de formule (III) :

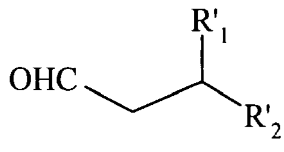

(III)

dans laquelle R'$_1$ et R'$_2$ forment ensemble la chaîne carbonée suivante :

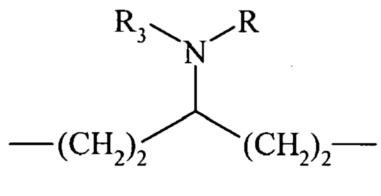

dans laquelle $R_3$ est tel que défini dans la revendication 1 et R représente un groupement protecteur de la fonction amine, comme par exemple le groupement *tert*-butyloxycarbonyle,
pour conduire au composé de formule (IV) :

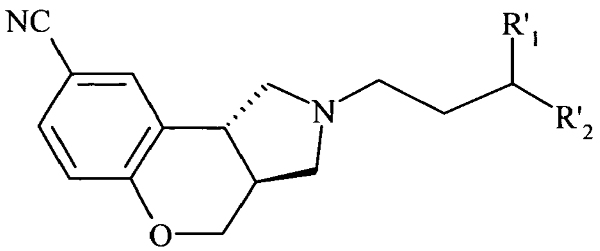

(IV)

dans laquelle R'$_1$ et R'$_2$ sont tels que définis précédemment,
qui est ensuite soumis à une réaction de déprotection de la fonction amine, par exemple en présence d'acide trifluoroacétique, pour conduire au composé de formule (I/a), cas particulier des composés de formule (I) :

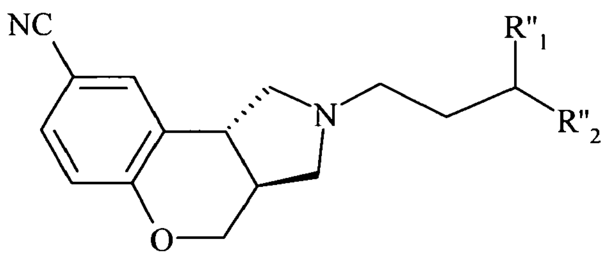

(I/a)

dans laquelle R"$_1$ et R"$_2$ forment ensemble la chaîne carbonée suivante :

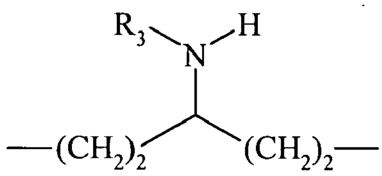

dans laquelle $R_3$ est tel que défini précédemment,
composé de formule (I/a) qui est ensuite soumis, si nécessaire :

■ **soit** à une réaction d'amination réductrice avec un agent réducteur, tel que le triacétoxyborohydrure ou le cyanoborohydrure de sodium, en présence d'un composé de formule (V) :

R'-CHO (V)

dans laquelle R' représente un atome d'hydrogène ou un groupement alkyle ($C_1$-$C_5$) linéaire ou ramifié ;
• **soit** à l'action d'un composé de formule (VI) :

R"-Y (VI)

dans laquelle Y représente un atome d'halogène, un groupement hydroxy ou alkoxy ($C_1$-$C_6$) linéaire ou ramifié et R" représente un groupement aryle, hétéroaryle, 3,4-dioxocyclobutenyle, $-COR_5$ ou $-SO_2R_5$, $R_5$ est tel que défini dans la revendication 1,
pour conduire au composé de formule (I/b), cas particulier des composés de formule (I) :

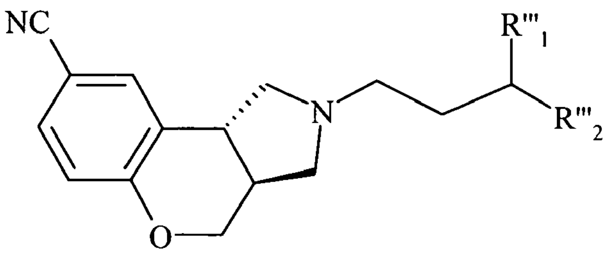 (I/b)

dans laquelle R"'$_1$ et R"'$_2$ forment ensemble la chaîne carbonée suivante :

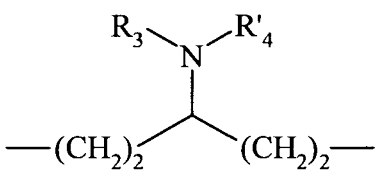

dans laquelle $R_3$ est tel que défini précédemment et $R'_4$ représente un groupement alkyle ($C_1$-$C_6$) linéaire ou ramifié, aryle, hétéroaryle, 3,4-dioxocyclobutenyle, $-COR_5$ ou $-SO_2R_5$, $R_5$ est tel que défini précédemment,
une variante dans la préparation du composé de formule (I/b) consistant, une fois l'étape de couplage sur le composé de formule (I/a) réalisée, en l'utilisation des réactions classiques de chimie afin de modifier, dans un deuxième temps, les substituants du composé de formule (VI),
les composés de formule (I/a) et (I/b), qui constituent l'ensemble des composés de formule (I), pouvant être ensuite purifiés selon une technique classique de séparation, que l'on transforme, si on le souhaite, en ses sels d'addition à un acide ou à une base pharmaceutiquement acceptable et dont on sépare éventuellement les isomères, s'ils

existent, selon une technique classique de séparation.

**13.** Compositions pharmaceutiques contenant comme principe actif un composé selon l'une quelconque des revendications 1 à 11, seul ou en combinaison avec un ou plusieurs excipients ou véhicules inertes, non toxiques et pharmaceutiquement acceptables.

**14.** Compositions pharmaceutiques selon la revendication 13 utiles pour le traitement ou la prévention de la schizophrénie et d'autres psychoses, de l'abus de drogues, des troubles provoqués par le stress comme les états anxieux et la toxicomanie, le traitement des états dépressifs, unipolaires et bipolaires, des comportements impulsifs tels que les troubles obsessifs compulsifs et l'agressivité, le traitement de la maladie de Parkinson, du tremblement essentiel, des troubles de la mémoire et d'autres troubles cognitifs associés aux maladies psychiatriques et neurologiques comme les démences et la maladie d'Alzheimer, des troubles développementaux chez l'enfant ou l'adolescent, le traitement de la douleur, des nausées, de l'éjaculation prématurée ainsi que pour la protection rénale.

**15.** Utilisation des composés de formule (I) selon l'une quelconque des revendications 1 à 11 pour la fabrication de médicaments utiles pour le traitement ou la prévention de la schizophrénie et d'autres psychoses, de l'abus de drogues, des troubles provoqués par le stress comme les états anxieux et la toxicomanie, le traitement des états dépressifs, unipolaires et bipolaires, des comportements impulsifs tels que les troubles obsessifs compulsifs et l'agressivité, le traitement de la maladie de Parkinson, du tremblement essentiel, des troubles de la mémoire et d'autres troubles cognitifs associés aux maladies psychiatriques et neurologiques comme les démences et la maladie d'Alzheimer, des troubles développementaux chez l'enfant ou l'adolescent, le traitement de la douleur, des nausées, de l'éjaculation prématurée ainsi que pour la protection rénale.

**16.** Composés de formule (I) selon l'une quelconque des revendications 1 à 11 utiles pour le traitement ou la prévention de la schizophrénie et d'autres psychoses, de l'abus de drogues, des troubles provoqués par le stress comme les états anxieux et la toxicomanie, le traitement des états dépressifs, unipolaires et bipolaires, des comportements impulsifs tels que les troubles obsessifs compulsifs et l'agressivité, le traitement de la maladie de Parkinson, du tremblement essentiel, des troubles de la mémoire et d'autres troubles cognitifs associés aux maladies psychiatriques et neurologiques comme les démences et la maladie d'Alzheimer, des troubles développementaux chez l'enfant ou l'adolescent, le traitement de la douleur, des nausées, de l'éjaculation prématurée ainsi que pour la protection rénale.

Europäisches Patentamt
European Patent Office
Office européen des brevets

# RAPPORT DE RECHERCHE EUROPEENNE

Numéro de la demande
EP 10 29 0411

| DOCUMENTS CONSIDERES COMME PERTINENTS | | | |
|---|---|---|---|
| Catégorie | Citation du document avec indication, en cas de besoin, des parties pertinentes | Revendication concernée | CLASSEMENT DE LA DEMANDE (IPC) |
| Y | EP 0 887 350 A1 (ADIR [FR]) 30 décembre 1998 (1998-12-30) * le document en entier * ----- | 1-16 | INV. C07D491/04 A61K31/407 |
| Y | DUBUFFET, THIERRY ET AL: "Novel benzopyrano[3,4-c]pyrrole derivatives as potent and selective dopamine D3 receptor antagonists", BIOORGANIC & MEDICINAL CHEMISTRY LETTERS, vol. 9, no. 14, 1999, pages 2059-2064, XP002580469, * tableau II * ----- | 1-16 | |
| Y | MILLAN, MARK J. ET AL: "S33138 [N-4-[2-[(3aS,9bR)-8-cyano]-1,3a,4,9b-tetrahydro[1]benzopyrano[3,4-c]pyrrol-2(3H)-yl- ethyl]phenylacetamide], a preferential dopamine D3 versus D2 receptor antagonist and potential antipsychotic agent: I. Receptor-binding profile and functional actions at G-protein-coupled receptors", JOURNAL OF PHARMACOLOGY AND EXPERIMENTAL THERAPEUTICS, vol. 324, no. 2, 2008, pages 587-599, XP002609855, * tableau 1 * ----- -/-- | 1-16 | DOMAINES TECHNIQUES RECHERCHES (IPC): C07D A61K |

Le présent rapport a été établi pour toutes les revendications

1

| Lieu de la recherche | Date d'achèvement de la recherche | Examinateur |
|---|---|---|
| Berlin | 17 novembre 2010 | Frelon, Didier |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................
& : membre de la même famille, document correspondant

EPO FORM 1503 03.82 (P04C02)

Europäisches Patentamt
European Patent Office
Office européen des brevets

# RAPPORT DE RECHERCHE EUROPEENNE

Numéro de la demande
EP 10 29 0411

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | MILLAN, MARK J. ET AL: "S33138 [N-[4-[2-((3aS,9bR)-8-cyano-1,3a,4,9b-tetrahydro[1]benzopyrano[3,4-c]pyrrol-2(3H)- yl)ethyl]phenylacetamide]], a preferential dopamine D3 versus D2 receptor antagonist and potential antipsychotic agent. II. A neurochemical, electrophysiological and behavioral characterization in vivo", JOURNAL OF PHARMACOLOGY AND EXPERIMENTAL THERAPEUTICS, vol. 324, no. 2, 2008, pages 600-611, XP002609856, * abrégé * ----- | 1-16 | |
| A | MILLAN, MARK J. ET AL: "S33138 (N-[4-[2-[((3aS,9bR)-8-cyano-1,3a,4,9b-tetrahydro[1]benzopyrano[3,4-c]pyrrol-2(3H)- yl)ethyl]phenylacetamide]]), a preferential dopamine D3 versus D2 receptor antagonist and potential antipsychotic agent: III. Actions in models of therapeutic activity and induction of side effects", JOURNAL OF PHARMACOLOGY AND EXPERIMENTAL THERAPEUTICS, vol. 324, no. 3, 2008, pages 1212-1226, XP002580473, * abrégé * ----- -/-- | 1-16 | DOMAINES TECHNIQUES RECHERCHES (IPC) |

Le présent rapport a été établi pour toutes les revendications

1

| Lieu de la recherche | Date d'achèvement de la recherche | Examinateur |
|---|---|---|
| Berlin | 17 novembre 2010 | Frelon, Didier |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Europäisches Patentamt
European Patent Office
Office européen des brevets

## RAPPORT DE RECHERCHE EUROPEENNE

Numéro de la demande
EP 10 29 0411

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | MILLAN, MARK J. ET AL: "S33084, a novel, potent, selective, and competitive antagonist at dopamine D3-receptors: II. Functional and behavioral profile compared with GR218,231 and L741,626", JOURNAL OF PHARMACOLOGY AND EXPERIMENTAL THERAPEUTICS, vol. 293, no. 3, 2000, pages 1063-1073, XP008121989, * abrégé * ----- | 1-16 | |
| A | YARKOV, ALEX V. ET AL: "Behavioral effects of dopamine agonists and antagonists in MPTP-lesioned D3 receptor knockout mice", PHARMACOLOGY, BIOCHEMISTRY AND BEHAVIOR, vol. 76, no. 3-4, 2003, pages 551-562, XP002580471, * abrégé * ----- | 1-16 | |
| A | NOVI, FRANCESCA ET AL: "Partial agonist actions of aripiprazole and the candidate antipsychotics S33592, bifeprunox, N-desmethylclozapine and preclamol at dopamine D2L receptors are modified by co-transfection of D3 receptors: potential role of heterodimer formation", JOURNAL OF NEUROCHEMISTRY, vol. 102, no. 4, 2007, pages 1410-1424, XP002580472, * abrégé * ----- | 1-16 | DOMAINES TECHNIQUES RECHERCHES (IPC) |

Le présent rapport a été établi pour toutes les revendications

1

| Lieu de la recherche | Date d'achèvement de la recherche | Examinateur |
|---|---|---|
| Berlin | 17 novembre 2010 | Frelon, Didier |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
........................................
& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 10 29 0411

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

17-11-2010

| Document brevet cité au rapport de recherche | Date de publication | Membre(s) de la famille de brevet(s) | Date de publication |
|---|---|---|---|
| EP 0887350 A1 | 30-12-1998 | AT 204578 T | 15-09-2001 |
| | | AU 735062 B2 | 28-06-2001 |
| | | AU 7314098 A | 07-01-1999 |
| | | BR 9802212 A | 25-05-1999 |
| | | CA 2242015 A1 | 24-12-1998 |
| | | CN 1203235 A | 30-12-1998 |
| | | DE 69801400 D1 | 27-09-2001 |
| | | DE 69801400 T2 | 16-05-2002 |
| | | DK 887350 T3 | 08-10-2001 |
| | | ES 2163239 T3 | 16-01-2002 |
| | | FR 2764890 A1 | 24-12-1998 |
| | | GR 3037038 T3 | 31-01-2002 |
| | | HK 1016987 A1 | 27-06-2003 |
| | | HU 9801423 A2 | 29-03-1999 |
| | | JP 4065604 B2 | 26-03-2008 |
| | | JP 11071376 A | 16-03-1999 |
| | | NO 982921 A | 28-12-1998 |
| | | NZ 330787 A | 29-04-1999 |
| | | PL 327002 A1 | 04-01-1999 |
| | | PT 887350 E | 30-11-2001 |
| | | US 6090837 A | 18-07-2000 |
| | | ZA 9805501 A | 20-01-1999 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 20050020645 A


### Littérature non-brevet citée dans la description


- *Psychopharmacology,* 1998, vol. 135, 1-16
- *CNS Neurol. Disord. Drug Targets,* 2006, vol. 5, 25-43
- *CNS Drug Discov.,* 2006, vol. 1, 271-88
- *Drug Discov. Today,* 2005, vol. 10, 917-25
- *Thérapie,* 2008, vol. 63, 187-229
- *Neurosci. Behav. Rev.,* 2001, vol. 25, 427-43
- *Brain Res. Rev.,* 2005, vol. 49, 77-105
- *J. Med. Chem.,* 2005, vol. 48, 3664-79
- *Int. J. Neuropsychopharmacol.,* 2007, vol. 10, 167-81
- *J. Pharmacol. Exp. Ther.,* 2007, vol. 321, 573-82
- *Neuropsychopharmacol.,* 2003, vol. 28, 1272-80
- *Int. J. Neuropsychopharmacol.,* 2006, vol. 9, 585-602
- *Synapse,* 2003, vol. 48, 154-6
- *Psychopharmacology,* 2004, vol. 175, 127-33
- *Pharmacol. Biochem. Behav.,* 2005, vol. 81, 190-7
- *FASEB J.,* 2007, vol. 20, 2223-33
- *Psychopharmacology,* 2004, vol. 176, 57-65
- *Prog. Neurobiol.,* 2003, vol. 70, 83-244
- *Eur. Neuropsychopharmacol.,* 2008, vol. 18, 271-7
- *Mol. Interv.,* 2008, vol. 8, 230-41
- *Psychiatry Res.,* 2003, vol. 119, 1-10
- *Am. J. Med. Genet. B Neuropsychiatr. Genet.,* 2006, vol. 141B, 409-13
- *J. Neural. Transm.,* 2003, vol. 110, 561-72
- *Neurobiol. Dis.,* 2009
- *Exp. Neurol.,* 2004, vol. 188, 128-38
- *PNAS,* 2006, vol. 103, 10753-8
- *Brain,* 2007, vol. 130, 1456-64
- *Psychopharmacology,* 2005, vol. 179, 567-75
- *J. Neurochem.,* 2007, vol. 100, 1047-61
- *Biol. Psychiatry,* 2008, vol. 65, 625-30
- *Psychopharmacology,* 1999, vol. 144, 239-47
- *Prog. Neurobiol.,* 2002, vol. 66, 355-474
- *J. Neural Transm.,* 1999, vol. 105, 1045-61
- *Eur. J. Pharmacol.,* 1996, vol. 301, 143-9
- *J. Sex. Med.,* 2009, vol. 6, 980-8
- *Br. J. Pharmacol.,* 2008, vol. 154, 1150-9
- *Lab. Investigation,* 2006, vol. 86, 262-74
- *Naunyn Schmiedebergs Arch. Pharmacol.,* 2005, vol. 371, 420-7
- *Bioorg. Med. Chem. Lett.,* 1999, vol. 9, 2059-2064
- *J. Org. Chem.,* 1989, vol. 54, 389-393
- *Biochemistry,* 2001, vol. 40, 5226-5232
- *Synthetic Communications,* 2005, vol. 35, 657-662